# FASCICULE DE BREVET EUROPEEN

(11) **EP 1 016 380 B1**
(45) Date de publication et mention de la délivrance du brevet: **03.11.2004**
(21) Numéro de dépôt: 99811168.6
(22) Date de dépôt: 17.12.1999
(51) Int. Cl.: A61B 17/62

(54) **Dispositif de positionnement et de blocage, notamment pour fixateurs externes**
Vorrichtung zum Positionieren und Verriegeln, insbesondere für Aussenfixateure
Positioning and locking device, particularly for external fixators

(30) Priorité: 29.12.1998 CH 258298
(43) Date de publication de la demande: 05.07.2000
(73) Titulaire: Stryker Trauma SA, 2545 Selzach (CH)
(72) Inventeur: Mata, Jacques, 1163 Etoy (CH)
(74) Mandataire: Isler & Pedrazzini AG

(56) Documents cités:
- EP-A- 0 700 664
- EP-A- 0 704 192
- EP-A- 0 813 845
- WO-A-97/35527

## Description

La présente invention a pour objet un dispositif de positionnement et de blocage pour des barres, des fiches ou des fils, notamment pour fixateur externe dans le domaine de l'orthopédie, spécialement utilisé comme pièce support en ostéosynthèse et ostéoplastie pour être incorporé dans des éléments fixateurs externes lors de la fracture osseuse.

Dans le marché on trouve grand nombre de pièces support pour permettre des assemblages afin de répondre aux besoins chaque jour plus exigeants de la chirurgie orthopédique. Il convient de sélectionner celles qui sont plus aptes à remplir leur fonction de par leur simplicité et leur maniabilité, le temps étant un facteur très important lors de ces opérations. Si les fixateurs externes existent depuis un certain temps, il y a toujours une évolution dans les pièces support afin de proposer des solutions plus performantes aux problèmes de chaque jour.

Le brevet européen EP 0 190 990 a pour objet un élément d'arceau et également un fixateur externe dans lequel est incorporé cet élément d'arceau avec les organes de fixation de fiches et de fils, destiné à être utilisé en ostéosynthèse et ostéoplastie.

Le PCT n° 97/35 527 décrit un dispositif de serrage présentant deux mâchoires placées sous l'action d'un ressort. Les mâchoires peuvent retenir un cadre sous l'action d'un ressort avant d'être bloquées au moyen d'un écrou. La partie supérieure du dispositif permet de bloquer un fils. Cependant, le cadre maintenu par les mâchoires ou le fils doivent être mis en place avant d'être serrés et bloqués par le dispositif. En aucun cas ces pièces ne peuvent être introduites par clipsage dans les mâchoires contre l'action d'un ressort.

La demande de brevet européen EP 0 700 664 A1 nous présente une articulation pour composants d'un fixateur externe pour le positionnement relatif de barres de fixation ou de fiches, et qui comporte deux paires de mâchoires agencées de manière à ce que les moyens élastiques sont intercalés entre les mâchoires et pressent les faces adjacentes desdites mâchoires pour arriver à un blocage de l'articulation. Les mâchoires présentant dans leurs faces adjacentes des rainures qui sont positionnées et agencées de manière à présenter une ouverture permettant le clipsage d'une barre ou d'une fiche par pression.

Les dispositifs de maintien et de serrage connus dans l'art antérieur présentent le désavantage de ne pas se maintenir seuls dans une position choisie lors de leur mise en place.

L'utilisateur est donc dans l'obligation de maintenir lui-même le positionnement relatif des barres, fiches et fils, jusqu'au moment où il effectue l'opération de serrage ou de blocage.

Le but de la présente invention est de remédier à cet inconvénient et de proposer un dispositif qui est maintenu par lui-même dans sa position d'assemblage, qui peut être modifiée, jusqu'au moment du serrage ou du blocage de l'ensemble, tout en restant un dispositif simple composé de deux mâchoires et d'un élément ressort.

La présente invention correspond à un dispositif de positionnement et de blocage pour des barres, des fiches ou des avec les caractéristiques suivant les revendications 1, 11 ou 16.

Selon le mode d'exécution suivant la revendication 1, l'élément de liaison est une douille traversant les deux mors et permettant le placement à son intérieur d'une barre retenue agencée pour être bloquée avec les mors par l'organe de serrage. L'organe élastique est un ressort se présentant sous forme d'agrafe reliant les deux mors, à la partie opposée à la zone d'enclipsage; il peut être simple ou double. Les extrémités libres de l'agrafe formant le ressort sont introduites dans des orifices pratiqués dans les mors. Selon un autre mode d'exécution l'élément élastique est un ressort axial de traction.

Dans les deux modes d'exécution les deux mors disposent d'encoches d'assemblage qui permettent leur positionnement relatif. Les mors et la douille présentent des dispositifs qui empêchent la rotation des uns par rapport aux autres. Le mors supérieur présente une creusure conique et un alésage cylindrique pour recevoir la douille, le mors inférieur présente un alésage qui a deux surfaces plates opposées pour collaborer à l'emboîtement de la douille, celle-ci ayant dans sa partie centrale deux surfaces plates opposées dans le sens de son axe qui se correspondent avec celles du mors inférieur pour faciliter son assemblage à l'intérieur dudit mors inférieur.

La douille, qui est cylindrique, présente une partie supérieure sous forme de cône tronqué avec des rainures dans le sens de son axe pour permettre le serrage de la barre, une fois qu'elle est placée à son intérieur. La douille présente sa partie inférieure filetée avec un diamètre en rapport avec celui de l'écrou de serrage.

Selon le mode d'exécution suivant la revendication 11, l'élément de liaison est une tige traversant un anneau relié à une barre, le mors supérieur étant placé à une des extrémités de la tige, autour de laquelle se positionnent le mors inférieur et l'écrou de serrage.

Selon ce même mode d'exécution les deux mors présentent une ouverture sous forme de mâchoire avec au moins une cavité permettant le clipsage d'une fiche ou d'un fil. Les deux mors présentent des billes en matière dure placées en alternance entre les cavités pour aider au blocage du fil logé à son intérieur.

L'écrou est réalisé avantageusement sous forme d'un bouton cannelé vissé autour de la partie inférieure de la tige qui permet le maintient de tout le dispositif assemblé. Une couronne dentée est intercalée entre le bouton cannelé et le pilier pour fixer le positionnement angulaire du dispositif qui restera bloqué par la suite, une fois que le bouton cannelé est serré. Le dispositif comprend un ressort hélicoïdal axial entre l'anneau et la couronne dentée.

Selon le mode d'exécution suivant la revendication 16, l'élément de liaison se présente sous forme d'un boulon dont l'extrémité libre est fileté pour venir se visser dans un alésage du pilier, le boulon traversant une pince délimitant au moins une mâchoire se présentant sous forme d'une fente placée transversalement par rapport à l'élément de liaison, la matière placée entre le fond de la fente et la surface extérieure de la pince faisant office de ressort.

Selon l'un ou l'autre des modes d'exécution, le dispositif est apte à servir de pièce support d'un fixateur externe comportant un arceau, des fiches ou des fils, des barres dans lequel les dispositifs présentent des ouvertures extérieures permettant le clipsage de l'arceau, des fiches et de fils ainsi que la fixation des barres ou desdits dispositifs par leurs piliers pour obtenir par serrage des dispositifs, leur positionnement relatif avant le blocage.

Le dessin illustre, à titre d'exemples, des modes de réalisation du dispositif avec mâchoire d'enclipsage, selon l'invention.

Dans le dessin,
la figure 1 montre une vue en perspective d'un mode d'exécution du dispositif selon une première manière de réalisation,
la figure 2 montre une coupe à travers le dispositif de la figure 1,
la figure 3 montre une coupe du dispositif de la figure 1 avec une variante d'un organe élastique,
la figure 4 est une vue en perspective d'un autre mode de réalisation du dispositif,
la figure 5 montre une coupe à travers le dispositif de la figure 3,
les figures 6 et 7 montrent le dispositif de la figure 3 maintenant dans sa mâchoire une fiche et un fil respectivement,
la figure 8 montre une coupe du dispositif selon un troisième mode de réalisation, et
la figure 9 montre une partie d'un fixateur externe pour ostéosynthèse et ostéoplastie dans lequel une série de dispositifs avec mâchoire d'enclipsage sont présentés comme pièces support pour le positionnement de barres, de fiches et de fils en collaboration avec un arceau.

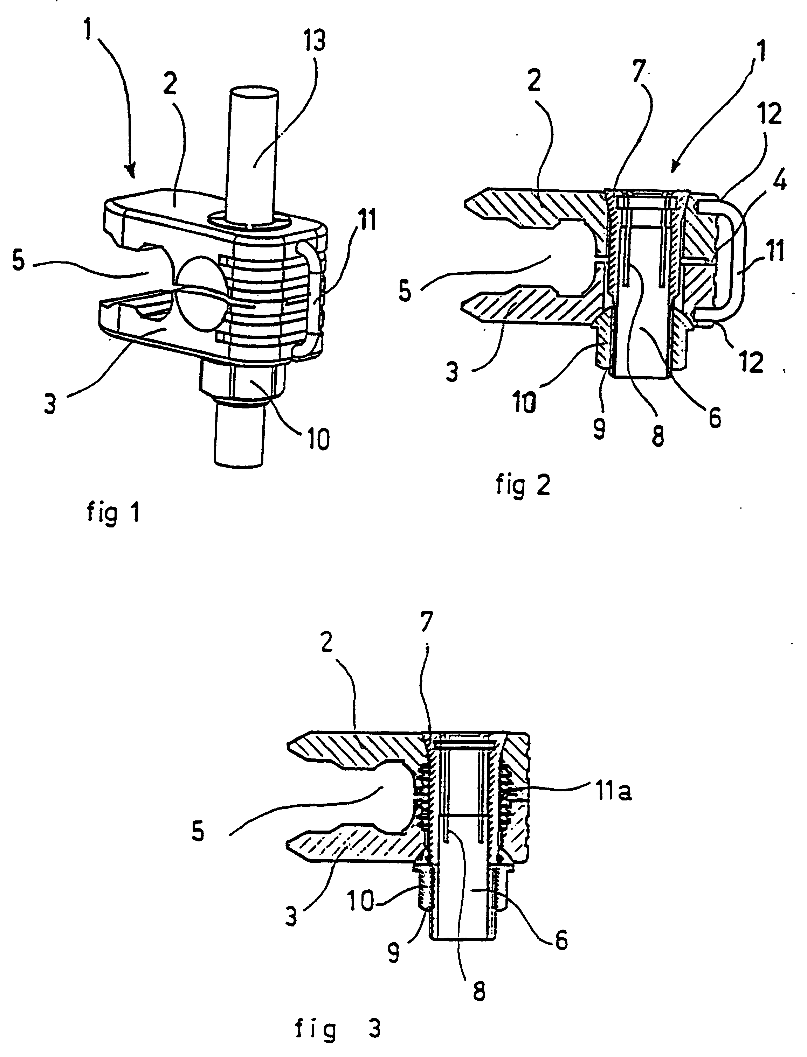

Dans les figures 1 et 2 du dessin, le dispositif de positionnement et de blocage 1 se compose d'un mors supérieur 2 et un mors inférieur 3 usinés avec des encoches d'assemblage 4 pour permettre leur positionnement de l'un contre l'autre, laissant à l'extrémité opposée une ouverture 5. Une douille 6 traverse perpendiculairement les deux mors 2,3 par l'extrémité opposée à l'ouverture de la mâchoire et présente dans sa partie supérieure 7 une forme de tronc de cône avec de rainures 8 qui partent de cette partie supérieure 7 et qui vont dans le sens de l'axe la douille pour se fermer vers la moitié de la longueur de celle-ci. Sa partie inférieure étant filetée en 9, est vissée à un écrou 10, qui serre les deux mors 2,3. Un ressort sous forme d'agrafe 11 va se loger à l'intérieur de deux trous 12 pratiqués chacun dans un des mors 2,3 étant aussi du côté opposé à l'ouverture de la mâchoire. Le mors supérieur 2 présente une creusure conique dans sa partie supérieure et un alésage cylindrique pour recevoir la douille 6 et le mors inférieur 3 présente un alésage qui a deux surfaces plates opposées pour collaborer à l'emboîtement de la douille 6, ces surfaces plates n'étant pas représentées dans le dessin. La douille 6, à son tour, a un diamètre plus petit dans sa partie inférieure filetée 7 et dans sa partie centrale dispose de deux surfaces plates opposées, non représentées dans le dessin, pour empêcher la rotation de la douille relativement aux mors et permettre le blocage de l'écrou.

Entre la douille 6 et les alésages des mors 2,3 existe un jeu permettant l'enclipsage du dispositif 1 avec la collaboration de l'agrafe 11, avant l'opération de serrage. En effet, le jeu permet la séparation de mors 2,3 juste pour enclipsage, par exemple sur une barre ou sur un arceau au long duquel le dispositif 1 peut coulisser et par conséquent changer de position. La barre 13 introduite dans la douille 6 reste en revanche fixée sur une position concrète d'assemblage de manière à ce qu'une personne puisse par elle-même régler les positions relatives de tous les éléments du dispositif avant l'opération de serrage final de l'ensemble.

La figure 3 montre en coupe le dispositif des figures 1 et 2 dans lequel l'organe élastique est maintenant un ressort axial de traction 11a qui dans sa position d'équilibre maintient les deux mors 2,3 assemblés, leur permettant de s'ouvrir pendant l'opération de clipsage et tenant le dispositif bloqué jusqu'au serrage final à l'aide de l'écrou 10.

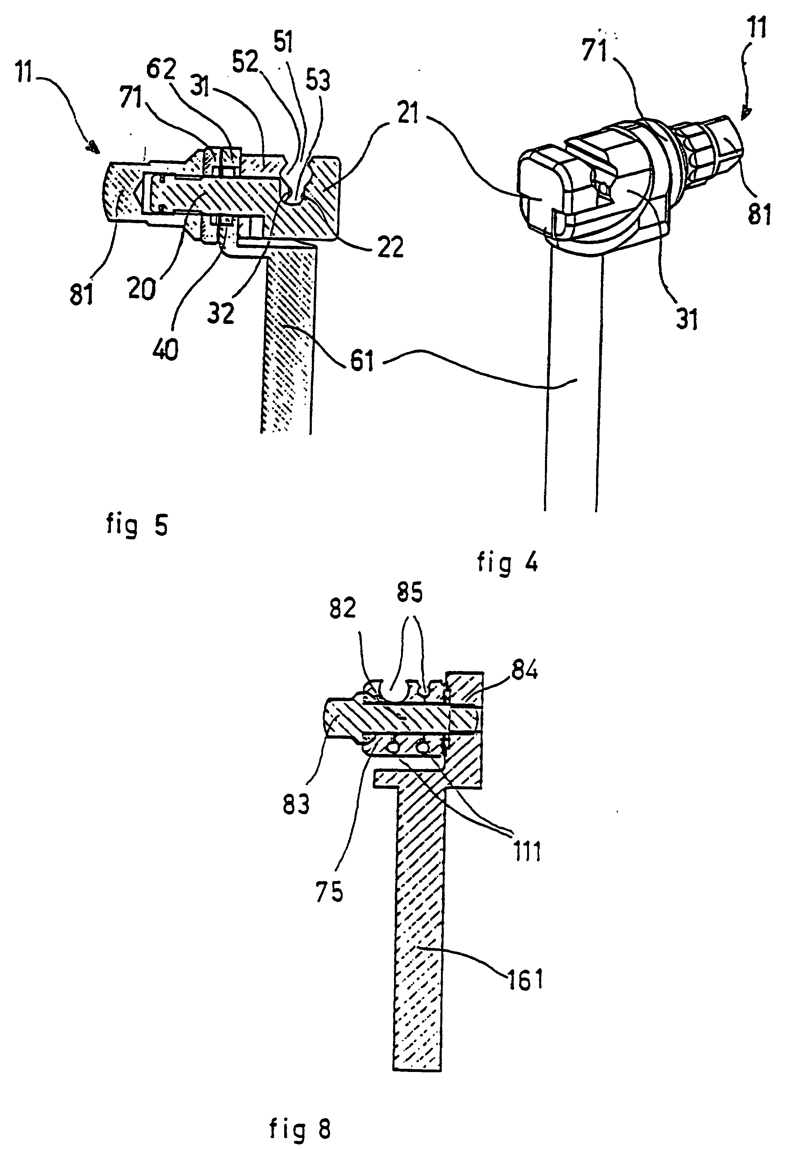

Les figures 4 à 7 montrent un autre mode d'exécution dans lequel le dispositif 11 se compose d'un tirant 20 qui présente à une de ses extrémités un mors supérieur 21 avec des billes 22 en une matière plus dure que la pièce pincée, réalisées par exemple en céramique ou en un métal dur; autour du tirant 20 se placent successivement un mors inférieur 31 avec d'autres billes 32 en toute matière plus dure que la pièce à maintenir, les deux mors 21,31 créant une ouverture extérieure 51 à deux cavités 52,53 ; un ressort de spirale 40, un pilier 61 terminé en anneau 62 à une de ses extrémités, une plaque dentée 71 et un écrou en forme de bouton cannelé 81. Le dessin montre comment le dispositif 11 clipse une fiche 16 dans sa cavité extérieure 52 ou un fil 17 dans sa cavité intérieure 53.

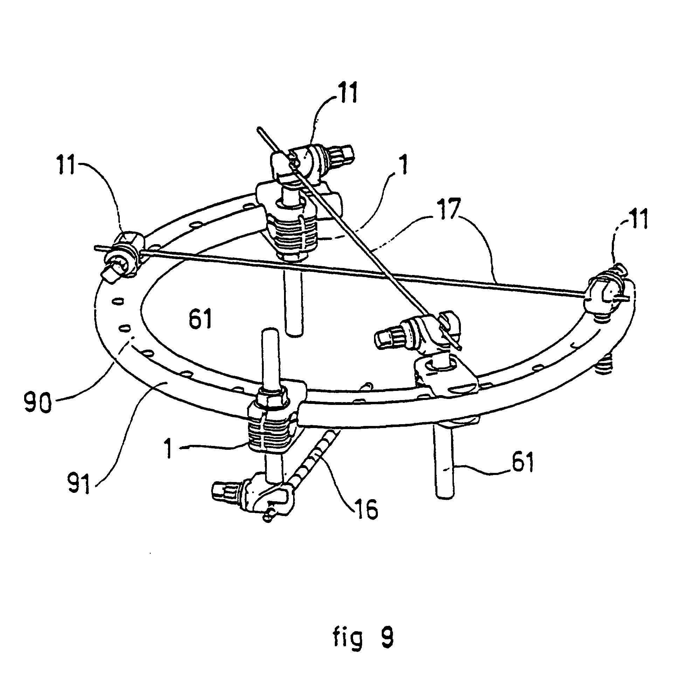

La figure 8 présente un troisième mode d'exécution avec comme élément de liaison un écrou 82. La pièce 75 est une pince présentant des jeux de mâchoires avec des ouvertures 85, dans lesquelles peuvent être enclipsés des fiches ou des fils. L'élément élastique est incorporé dans la pince et correspond à la partie de matériel mince située à la partie opposée des ouvertures et faisant office de ressort.

Selon le premier mode d'exécution, la douille 6 qui traverse les deux mors 2,3, est ajustée à l'intérieur du mors supérieur 2 à l'aide de sa forme de tronc de cône de sa partie supérieure, qui va s'épauler contre la creusure de la même forme du mors supérieur. Une barre 13 est introduite à l'intérieur de la douille 6 pour être finalement serrée à l'aide de l'écrou 10. Celui-la dispose d'une large tête pour faciliter le serrage manuel avant d'être bloqué au moyen de tout outil adéquat lorsque le système est en place. Le dispositif 1 avec la barre 13 est clipsé par son ouverture extérieure 5 par pression sur un arceau et reste dans cette position grâce à la collaboration du ressort 11,11a. Il va sans dire que toute autre forme d'ouverture est envisageable sans sortir du cadre de la présente invention, pour permettre le clipsage du dispositif sur toute autre pièce non représentée.

Selon le second mode d'exécution les deux mors 21,31 clipsent par pression une fiche 16 ou un fil 17 respectivement dans les cavités extérieure 52 ou intérieure 53, avec la collaboration du ressort 40. Celui-ci est un ressort en spirale, qui s'appuyant contre le bouton cannelé 101 presse le mors inférieur 31 contre le mors supérieur 21 ; les billes 22,32 placés avec un décalage retiennent le fil de façon que celui-ci ne puisse échapper de la mâchoire. Le bouton cannelé 101 presse l'ensemble dispositif-fiche ou dispositif-fil et une fois que le pilier 61, qui dispose d'une extrémité sous forme d'anneau 62, se trouve orienté dans sa position finale, le serre contre la plaque dentée 71 pour qu'il reste bloqué. Ledit bouton cannelé 101 présente une tête carrée, mais il va sans dire que toute autre forme de tête est envisageable sans sortir du cadre de la présente invention. Comme dans la première forme de réalisation et pour la même raison, il dispose d'une large tête pour faciliter un premier serrage manuel avant le blocage final lorsque le système est en place.

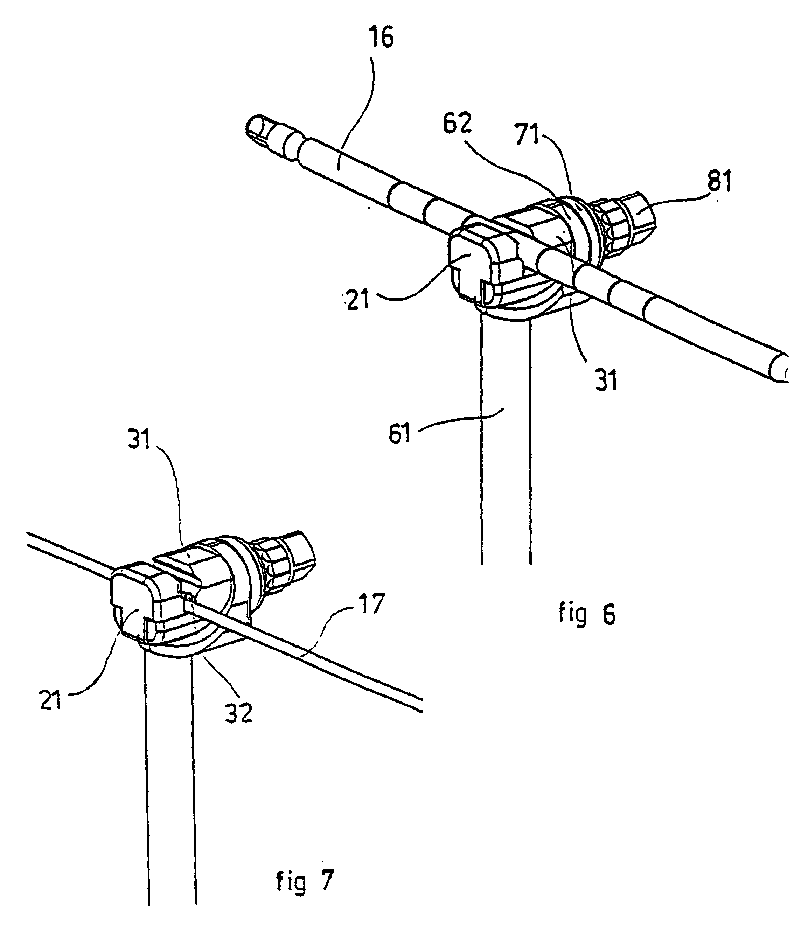

Dans la figure 9 est montré un élément d'arceau 91, sans qu'un cercle complet ne soit formé, qui fait partie d'un fixateur externe 90. L'élément d'arceau dispose d'une section avec une âme au centre de forme générale rectangulaire qui sert d'élément de liaison aux deux renforts latéraux de section symétrique par rapport à un axe perpendiculaire à l'âme permettant aux pièces support d'être clipsées à l'intérieur ou à l'extérieur de l'arceau selon les besoins. Cette figure présente trois dispositifs avec mâchoires d'enclipsage selon la première manière de réalisation 1 qui sont clipsés en deux occasions à l'intérieur de l'arceau et dans une troisième à son extérieur. Dans les trois cas, les dispositifs 1 sont assemblés avec d'autres dispositifs 11 selon la deuxième forme de réalisation pour montrer comment les deux formes de réalisation 1,11 selon l'invention peuvent collaborer ensemble, le pilier 61 de l'un devenant la barre 13 de l'autre. Deux paires de dispositifs 11 supportent deux fils transfixiants qui traversent un os non représenté dans la figure, de part en part, un troisième tient une fiche 16 qui serait également introduite dans une autre partie de l'os. La combinaison de tous ces éléments, arceau avec des fiches, des fils, des barres et des dispositifs avec mâchoire d'enclipsage permet de créer un système performant pour obtenir le positionnement relatif des uns par rapport aux autres en le serrant manuellement pour procéder ensuite au blocage définitif en maintenant les positions relatives entre les différents éléments. Le dispositif de la figure 7 peut également être assemblé à l'aide d'un deuxième arceau 91.

## Revendications

1. Dispositif de positionnement et de blocage des barres, des fiches ou des fils, notamment pour fixateur externe dans le domaine de l'orthopédie comprenant un élément de liaison (6, 20, 82) solidaire d'un organe de serrage (10, 81, 83) et deux mors libres qui forment aux moins une ouverture (5,51, 85) présentant au moins une cavité sous forme de mâchoire permettant le clipsage par pression d'un arceau, d'une barre, d'une fiche ou d'un fil, contre l'action d'un élément élastique (11, 40, 111) maintenant le dispositif en position avant l'action de blocage effectuée par l'organe de serrage (10, 81, 83), **caractérisé en ce que** l'élément de liaison est une douille (6) traversant les deux mors (2, 3) et permettant le placement à son intérieur d'une barre (13) agencée pour être bloquée avec les mors par l'organe de serrage (10).

2. Dispositif selon la revendication 1, **caractérisé en ce que** l'élément élastique est un ressort (11) reliant les deux mors (2, 3).

3. Dispositif selon la revendication 2, **caractérisé en ce que** le ressort (11) se présente sous forme d'aux moins une agrafe, à la partie opposée de la cavité d'enclipsage (5).

4. Dispositif selon la revendication 3, **caractérisé en ce que** les extrémités libres des agrafes formant le ressort sont introduites dans des orifices (12) pratiqués dans les mors.

5. Dispositif selon la revendication 1, **caractérisé en ce que** l'élément élastique est un ressort axial (11a) de traction, placé autour de l'élément de liaison (6).

6. Dispositif selon la revendication 1, **caractérisé en ce que** les deux mors (2, 3) disposent d'encoches d'assemblage (4) qui permettent leur positionnement relatif.

7. Dispositif selon la revendication 1, **caractérisé en ce que** les deux mors (2, 3) et la douille (6) présentent des organes d'emboîtage qui empêchent la rotation des uns par rapport aux autres.

8. Dispositif selon la revendication 1, **caractérisé en ce que** le mors supérieur (2) présente une creusure conique et un alésage cylindrique pour recevoir la douille (6), le mors inférieur (3) présentant un alésage qui a deux surfaces opposées pour collaborer à l'emboîtement de la douille (6), celle-ci ayant dans sa partie centrale deux surfaces plates opposées dans le sens de son axe qui se correspondent avec celles du mors inférieur (3), pour faciliter son assemblage à l'intérieur dudit mors inférieur (3).

9. Dispositif selon la revendication 1, **caractérisé en ce que** la douille (6) à sa partie supérieure (7) se présente sous forme de tronc de cône avec des rainures (8) dans le sens de son axe pour permettre le serrage de la barre (13) une fois placée à son intérieur.

10. Dispositif selon la revendication 1, **caractérisé en ce que** la douille (6) présente une partie inférieure filetée destinée à recevoir l'organe de serrage sous forme d'écrou de serrage (10).

11. Dispositif de positionnement et de blocage pour des barres, des fiches ou des fils, notamment pour fixateur externe dans le domaine de l'orthopédie comprenant un élément de liaison (6, 20, 82) solidaire d'un organe de serrage (10, 81, 83) et deux mors libres qui forment aux moins une ouverture (5, 51, 85) présentant au moins une cavité sous forme de mâchoire permettent le clipsage par pression d'un arceau, d'une barre, d'une fiche ou d'un fil, contre l'action d'un élément élastique (11, 40, 111) maintenant le dispositif en position avant l'action de blocage effectuée par l'organe de serrage (10, 81, 83), **caractérisé en ce que** l'élément de liaison est une tige (20) traversant un anneau (62) relié à une barre (61), le mors supérieur (21) étant placé à une des extrémités de la tige (20), autour de laquelle se positionnent le mors inférieur (31) et l'organe de serrage sous forme d'écrou de serrage (81) et l'élément élastique (40).

12. Dispositif selon la revendication 11, **caractérisé en ce que** les deux mors (21, 31) présentent une cavité (51) avec une partie inférieure (53) et une partie supérieure (52) de plus grande dimension permettant le clipsage d'une fiche ou d'un fil

13. Dispositif selon la revendication 11, **caractérisé en ce qu'**il présente sur les deux mors (21, 31) des billes en matière dure (22, 32) placées entre la partie inférieure (53) et la partie supérieure (52) de la cavité (51) , pour aider au blocage du fil (17) logé dans une cavité intérieure (53) de la mâchoire, la partie supérieure (52) des deux mors (51, 52) étant destinées à recevoir une fiche.

14. Dispositif selon la revendication 11, **caractérisé en ce que** l'écrou est un bouton cannelé (81) vissé autour de la partie intérieure de la tige (20), une couronne dentée (71) destinée à fixer le positionnement angulaire du dispositif (11) étant intercalée entre l'anneau (62) et le bouton cannelé (81).

15. Dispositif selon la revendication 14, **caractérisé en ce que** l'élément élastique est un ressort hélicoïdal (40) placé entre l'anneau (62) et la couronne dentée (71).

16. Dispositif de positionnement et de blocage pour des barres, des fiches ou des fils, notamment pour fixateur externe dans le domaine de l'orthopédie comprenant un élément de liaison (6, 20, 82) solidaire d'un organe de serrage (10, 81, 83) et deux mors libres qui forment aux moins une ouverture (5, 51, 85) présentant au moins une cavité sous forme de mâchoire permettant le clipsage par pression d'un arceau, d'une barre, d'une fiche ou d'un fil, contre l'action d'un élément élastique (11, 40, 111) maintenant le dispositif en position avant l'action de blocage effectuée par l'organe de serrage (10, 81, 83), **caractérisé en ce qu'**élément de liaison (82) se présente sous forme d'un boulon dont l'extrémité libre est fileté (84) pour venir se visser dans un alésage d'une barre (161), le boulon traversant une pince (75) délimitant au moins une mâchoire (85) se présentant sous forme d'une fente placée transversalement par rapport à l'élément de liaison (82), où l'élément élastique est constitué d'une matière (111) placée entre le fond de la fente et la surface extérieure de la pincé.

17. Dispositif selon la revendication 1, 11 ou 16, **caractérisé en ce qu'**il est placé sur un arceau (91), destiné à retenir des fiches, des fils et/ou des barres de manière à constituer un fixateur externe.

## Patentansprüche

1. Vorrichtung zum Positionieren und Blockieren von Befestigungsstangen, Steckverbindern oder Drähten, insbesondere für einen äusseren Fixateur im Bereich der Orthopädie, mit einem Verbindungselement (6, 20, 82), das fest mit einem Spannorgan (10, 81, 83) verbunden ist, und mit zwei freien Klemmbacken, die mindestens eine Öffnung (5, 51, 85) bilden, die mindestens einen Hohlraum in Form einer Backe aufweist, die das Einklemmen unter Ausübung von Druck eines Bügels, einer Befestigungsstange, eines Steckverbinders oder eines Drahtes entgegen der wirkung eines elastischen Elementes (11, 40, 111) erlaubt, welches die Vorrichtung vor der Blockierung durch das Spannorgan (10, 81, 83) an Ort und Stelle hält, **dadurch gekennzeichnet, dass** das Verbindungselement eine Hülse (6) ist, die die beiden Klemmbacken (2, 3) durchquert und die in ihrem Innern das Anordnen einer Befestigungsstange (13) ermöglicht, die so ausgestaltet ist, dass sie durch das Spannorgan (10) mit den Klemmbacken blockierbar ist.

2. Vorrichtung nach Anspruch 1, **dadurch gekennzeichnet, dass** das elastische Element eine Feder (11) ist, die die beiden Klemmbacken (2, 3) miteinander verbindet.

3. Vorrichtung nach Anspruch 2, **dadurch gekennzeichnet, dass** die Feder (11) die Form von mindestens einer Spange am gegenüberliegenden Bereich der Einklemmöffnung (5) hat.

4. Vorrichtung nach Anspruch 3, **dadurch gekennzeichnet, dass** die freien Enden der Spangen, die die Feder bilden, in Öffnungen (12) eingeführt werden, die in den Klemmbacken eingeschnitten sind.

5. Vorrichtung nach Anspruch 1, **dadurch gekennzeichnet, dass** das elastische Element eine axiale Zugfeder (11a) ist, die um das Verbindungselement (6) herum angeordnet ist.

6. Vorrichtung nach Anspruch 1, **dadurch gekennzeichnet, dass** die zwei Klemmbacken (2, 3) mit Montagekerben (4) versehen sind, die ihre relative Positionierung ermöglichen.

7. Vorrichtung nach Anspruch 1, **dadurch gekennzeichnet, dass** die zwei Klemmbacken (2, 3) und die Hülse (6) Fügeorgane umfassen, die ein Verdrehen gegeneinander verhindern.

8. Vorrichtung nach Anspruch 1, **dadurch gekennzeichnet, dass** die obere Klemmbacke (3) einen konischen Einschnitt und eine zylindrische Bohrung für die Aufnahme der Hülse (6) aufweist, und dass die untere Klemmbacke (2) mit einer Bohrung versehen ist, welche zwei gegenüberliegende ebene Flächen aufweist, um für das Einstecken der Hülse (6) zusammenzuwirken, welche in ihrem mittleren Abschnitt zwei ebene Flächen enthält, die sich in Richtung ihrer Achse gegenüberliegen, die denjenigen der unteren Klemmbacke (3) entsprechen, um den Einbau in das Innere der besagten unteren Klemmbacke (3) zu vereinfachen.

9. Vorrichtung nach Anspruch 1, **dadurch gekennzeichnet, dass** die Hülse (6) in ihrem oberen Teil (7) die Gestalt eines Stumpfkegels mit in Richtung ihrer Achse verlaufenden Kerben (8) aufweist, um das Verspannen der Befestigungsstange (13) zu gestatten, sobald sie in ihrem Innern angeordnet ist.

10. Vorrichtung nach Anspruch 1, **dadurch gekennzeichnet, dass** die Hülse (6) ein unteres mit einem Gewinde versehenes Ende aufweist, welches dazu vorgesehen ist, das Spannorgan in Gestalt einer Spannmutter (10) aufzunehmen.

11. Vorrichtung zum Positionieren und Blockieren von Befestigungsstangen, Steckverbindern oder Drähten, insbesondere für einen äusseren Fixateur im Bereich der Orthopädie, mit einem Verbindungselement (6, 20, 82), das fest mit einem Spannorgan (10, 81, 83) verbunden ist, und mit zwei freien Klemmbacken, die mindestens eine Öffnung (5, 51, 85) bilden, die mindestens einen Hohlraum in Form einer Backe aufweist, die das Einklemmen unter Ausübung von Druck eines Bügels, einer Befestigungsstange, eines Steckverbinders oder eines Drahtes entgegen der Wirkung eines elastischen Elementes (11, 40, 111) erlaubt, welches die Vorrichtung vor der Blockierung durch das Spannorgan (10, 81, 83) an Ort und Stelle hält, **dadurch gekennzeichnet, dass** das Verbindungselement eine Spindel (6) ist, welche einen an einer Befestigungsstange (61) befestigten Ring (62) durchquert, wobei die obere Klemmbacke (21) an einem Ende der Spindel (20) angeordnet ist, um die herum die untere Klemmbacke (31) und das Spannorgan in Gestalt einer Spannmutter (81) und das elastische Element (40) angeordnet sind.

12. Vorrichtung nach Anspruch 11, **dadurch gekennzeichnet, dass** die zwei Klemmbacken (21, 31) einen Hohlraum (51) mit einem unteren Abschnitt (53) und mit einem oberen Abschnitt (52) von grösserer Dimension aufweisen, welche das Einklemmen eines Steckverbinders oder eines Drahtes ermöglichen.

13. Vorrichtung nach Anspruch 11, **dadurch gekennzeichnet, dass** sie auf den beiden Klemmbacken (21, 31) Kugeln (22, 32) aus einem harten Material aufweist, die zwischen dem unteren Abschnitt (53) und dem oberen Abschnitt (52) des Hohlraums (51) angeordnet sind, um die Blockierung des Drahtes (17) zu unterstützen, der in einem unteren Hohlraum (53) der Backe angeordnet ist, wobei der obere Abschnitt (52) der beiden Klemmbacken (51, 52) zur Aufnahme eines Steckverbinders bestimmt ist.

14. Vorrichtung nach Anspruch 11, **dadurch gekennzeichnet, dass** die Mutter aus einem Rändelknopf (81) besteht, der um den unteren Abschnitt der Spindel (20) angeschraubt ist, und dass eine Zahnkrone (71), die dazu vorgesehen ist, die Winkelstellung der Vorrichtung (11) zu fixieren, zwischen dem Ring (62) und dem Rändelknopf (81) eingelegt ist.

15. Vorrichtung nach Anspruch 11, **dadurch gekennzeichnet, dass** das elastische Element eine Schraubenfeder (40) ist, die zwischen dem Ring (62) und dem Zahnkranz (71) angeordnet ist.

16. Vorrichtung zum Positionieren und Blockieren von Befestigungsstangen, Steckverbindern oder Drähten, insbesondere für einen äusseren Fixateur im Bereich der Orthopädie, mit einem Verbindungselement (6, 20, 82), das fest mit einem Spannorgan (10, 81, 83) verbunden ist, und mit zwei freien Klemmbacken, die mindestens eine Öffnung (5, 51, 85) bilden, die mindestens einen Hohlraum in Form einer Backe aufweist, die das Einklemmen unter Ausübung von Druck eines Bügels, einer Befestigungsstange, eines Steckverbinders oder eines Drahtes entgegen der Wirkung eines elastischen Elementes (11, 40, 111) erlaubt, welches die Vorrichtung vor der Blockierung durch das Spannorgan (10, 81, 83) an Ort und Stelle hält, **dadurch gekennzeichnet, dass** das Verbindungselement (82) die Form eines Bolzens aufweist, dessen freies Ende mit einem Gewinde (82) versehen ist, um in eine in einer Befestigungsstange (161) vorgesehene Bohrung einschraubbar zu sein, wobei der Bolzen eine Klemme (75) durchquert, die mindestens eine Klemmbacke (85) umgibt, die die Gestalt eines Schlitzes aufweist, der quer zu dem Verbindungselement (82) angeordnet ist, wobei das elastische Element aus einem Material (111) besteht, das zwischen dem Boden des Schlitzes und der äusseren Oberfläche der Klemme angeordnet ist.

17. Vorrichtung nach Anspruch 1, 11 oder 16, **dadurch gekennzeichnet, dass** sie auf einem Bügel (91) angeordnet ist, um Steckverbinder, Drähte und/oder Befestigungsstangen festzuhalten, so dass ein äusserer Fixateur hergestellt wird.

## Claims

1. A device for positioning and locking rods, pins or wires, particularly for an external fixator in the field of orthopedics, comprising a connecting element (6, 20, 82) secured to a tightening member (10, 81, 83) and two free jaw members forming at least one opening (5, 51, 85) exhibiting at least one cavity in the form of a gripping jaw allowing a hoop, a rod, a pin or a wire to be clipped by pressure against the action of an elastic means (11, 40, 111) which keeps the device in position prior to the locking action which is performed using the tightening member (10, 81, 83), **characterized in that** the connecting element is a bushing (6) which passes through the two jaws (2, 3) and permits the placement of a rod (13) in its interior, the rod being designed to be locked with the jaws by the tightening member (10) .

2. The device according to claim 1, wherein the elastic means is a spring (11) connecting the two jaws (2, 3).

3. The device according to claim 2, wherein the spring (11) is in the form of at least one staple at an end opposite of the clipping cavity (5).

4. The device according to claim 3, wherein the free ends of the staples forming the spring are introduced into orifices (12) made in the jaw members.

5. The device according to claim 1, wherein the elastic means is an axial tension spring (11a) mounted around the connecting element (6).

6. The device according to claim 1, wherein the two jaws (2, 3) have assembly notches (4) which position them relative to one another.

7. The device according to claim 1, wherein the two jaws (2, 3) and the bushing (6) have mating members which prevent one from rotating relative to another.

8. The device according to claim 1, wherein the upper jaw (2) has a conical recess and a cylindrical bore for accommodating the bushing (6), the lower jaw (3) having a bore which has two opposed flat surfaces to play a part in mating with the bushing (6), said bushing having two opposed flat surfaces in its central part in the direction of its axis which correspond with those of the lower jaw (3), simplifying the accommodation of said lower jaw (3) on its inside.

9. The device according to claim 1, wherein the bushing (6) has an upper part (7) in the form of a cone frustum with slots (8) in the direction of its axis to allow the rod (13) to be clamped tightly once it has been placed on its inside.

10. The device according to claim 1, wherein the bushing (6) has a lower threaded part provided to accommodate the tightening member in form of a tightening nut (10).

11. A device for positioning and locking rods, pins or wires, particularly for an external fixator in the field of orthopedics, comprising a connecting element (6, 20, 82) secured to a tightening member (10, 81, 83) and two free jaw members forming at least one opening (5, 51, 85) exhibiting at least one cavity in the form of a gripping jaw allowing a hoop, a rod, a pin or a wire to be clipped by pressure against the action of an elastic means (11, 40, 111) which keeps the device in position prior to the locking action which is performed using the tightening member (10, 81, 83), **characterized in that** the connecting element is a shank (20) passing through a ring (62) connected to a rod (61), the upper jaw member (21) being placed at one of the ends of the shank (20) around which the lower jaw (31) and the tightening member in form of a tightening nut (81) and the elastic means (40) are positioned.

12. The device according to claim 11, wherein the two jaws (21, 31) are in the form of a cavity (51) with a lower portion (53) and an upper portion (52) of a larger dimension allowing a pin or a wire to be clipped.

13. The device according to claim 11, wherein, on the two jaw members (21, 31), it comprises balls (22, 32) made of a hard material placed between the lower portion (53) and the upper portion (52) of the cavity (51) to assist with locking the wire (17) housed in a lower cavity (53) of the gripping jaw, the upper portion (52) of the two jaw members (51, 52) being intended to accommodate a pin.

14. The device according to claim 11, wherein the nut is a ribbed knob (81) screwed around the lower part of the shank (20), a toothed annulus (71) to set the angular position of the device (11) being inserted between the ring (62) and the ribbed knob (81).

15. The device according to claim 14, wherein the elastic means is a helical spring (40) placed between the ring (62) and the toothed annulus (71).

16. A device for positioning and locking rods, pins or wires, particularly for an external fixator in the field of orthopedics, comprising a connecting element (6, 20, 82) secured to a tightening member (10, 81, 83) and two free jaw members forming at least one opening (5, 51, 85) exhibiting at least one cavity in the form of a gripping jaw allowing a hoop, a rod, a pin or a wire to be clipped by pressure against the action of an elastic means (11, 40, 111) which keeps the device in position prior to the locking action which is performed using the tightening member (10, 81, 83), **characterized in that** the connecting element (82) is in the form of a bolt, the free end of which is threaded (84) to screw into a bore in a rod (161), the bolt passing through a clamp (75) delimiting at least one gripping jaw (85) in the form of a slot placed transversely with respect to the connecting element (82), wherein the elastic means is made of a material (111) placed between the bottom of the slot and the external surface of the clamp.

17. The device according to claim 1, 11 or 16, wherein it is placed on a hoop (91) intended to retain pins, wires and/or rods so as to form an external fixator.
